**Europäisches Patentamt**

**European Patent Office**

**Office européen des brevets**

(19)

(11) Veröffentlichungsnummer: **0 095 440**
**B1**

(12) **EUROPÄISCHE PATENTSCHRIFT**

(45) Veröffentlichungstag der Patentschrift:
**27.08.86**

(51) Int. Cl.⁴: **A 61 F 2/36**

(21) Anmeldenummer: **83730051.6**

(22) Anmeldetag: **24.05.83**

(54) **Kragenlose Hüftgelenkprothese.**

(30) Priorität: **21.05.82 DE 3219681**
**05.10.82 DE 3237203**

(43) Veröffentlichungstag der Anmeldung:
**30.11.83 Patentblatt 83/48**

(45) Bekanntmachung des Hinweises auf die Patenterteilung:
**27.08.86 Patentblatt 86/35**

(84) Benannte Vertragsstaaten:
**AT BE CH DE FR GB IT LI NL SE**

(56) Entgegenhaltungen:
**EP - A - 0 027 159**
**EP - A - 0 038 908**
**EP - A - 0 044 915**
**DE - A - 2 851 598**
**DE - B - 2 059 381**
**GB - A - 1 126 961**

(73) Patentinhaber: **MECRON MEDIZINISCHE PRODUKTE GMBH, Nunsdorfer Ring 25-27, D-1000 Berlin 48 (DE)**

(72) Erfinder: **Ecke, Hermann, Prof. Dr. med., Klinikstrasse 29, D-6300 Giessen (DE)**

(74) Vertreter: **Christiansen, Henning, Dipl.-Ing., Dietrich-Schäfer-Weg 21, D-1000 Berlin 41 (DE)**

**Beschreibung**

Die Erfindung betrifft eine kragenlose Hüftgelenkprothese der im Oberbegriff des Anspruchs 1 angegebenen Art zur zementlosen Verankerung.

Da sich in der Vergangenheit bei der Verwendung von Knochenzement gewisse Nachteile, wie z.B. Lockerungen, gezeigt haben, ist man bemüht, Endoprothesen möglichst ohne Verwendung von Knochenzement zu befestigen. Dabei treten jedoch ebenfalls eine Reihe von Schwierigkeiten auf. Wenn nämlich beispielsweise der Femurteil, welcher zur Aufnahme des Gelenkkopfes dient, einen «Kragen» aufweist, findet die Krafteinleitung in den Knochen ausschliesslich im Bereich des «überkragenden» Teiles statt. Da aber unter Belastung die bei diesen Verhältnissen in einem eng begrenzten Bereich auftretenden Kräfte relativ gross sind, kann es aufgrund der resultierenden «Wackelbewegungen» zur Lockerung des Prothesenschaftes kommen.

Weiterhin sind kragenlose Prothesen bekannt geworden, welche eine Verjüngung besitzen und sich im ausgebohrten Markraum des Femurs verkeilen. Eine derartige Prothese ist beispielsweise in der EP-A 0 027 159 beschrieben. Bei diesen Prothesen besteht der Nachteil, dass die durch die Verjüngung des Prothesenschaftes im distalen Teil bedingte Keilwirkung sehr gross ist, so dass die Knochenbeanspruchung in radialer Richtung unter Last erheblich ist und insbesondere beim osteoporotischen Knochen die Gefahr der Rissbildung besteht.

Wegen des komplizierten Verlaufs der sich aufweitenden Bereiche im äussersten proximalen Bereich des Schaftes ist es bei genauer Masshaltigkeit und normalen anatomischen Verhältnissen nur sehr schwer möglich, die Anpassung an den Markraum so zu gestalten, dass sich beim Einbringen des Schaftes eine definierte Kraftverteilung einstellt. Wenn die Prothese ausschliesslich in diesem Bereich trägt, kommt es wiederum zu einer mehr oder weniger punktuellen Belastung und den entsprechenden nachteiligen Eigenschaften für die Knochensubstanz.

Aus der EP-A 0 044 915 ist ein Schaftteil einer kragenlosen Hüftendoprothese bekanntgeworden, welcher einen eliptischen Querschnitt aufweist, wobei die Verkeilung im Bereich der dem Adambogen folgenden Abbiegung oder Krümmung des Prothesenschaftes erfolgt, so dass bei Belastung der Prothese vergleichsweise kleine Biegemomente wirksam werden und eine übermässige Beanspruchung des Knochengewebes ausgeschlossen sein soll. Auch bei dieser Prothese besteht aber der Nachteil, dass die Anpassung des Markraums an die relativ komplizierte Formgestaltung nur schwer durchführbar ist. Im übrigen muss die Knochenöffnung grossflächig ausgebildet sein, damit die Prothese einsetzbar ist, weil insbesondere der Bereich starker Krümmung mit elliptischem Querschnitt eine grössere Öffnung erfordert, als es dem der Gelenkkugel nahen Endquerschnitt der Prothese entspricht.

Weiterhin ist aus der DE-A 2 851 598 eine Prothese der eingangs genannten Gattung bekannt, bei der der Schaft in der AP-Ebene eine s-förmige Biegung aufweist. Diese Biegung bildet eine Anpassung an den natürlichen Verlauf des Markkanals in verhältnismässig grossem Abstand vom Hals und kann deshalb nur auf Prothesen mit relativ grosser Länge des Schafts angewendet werden. Da dem konvexen Bereich ein entsprechender konkaver Bereich auf der gegenüberliegenden Schaftseite entspricht, tritt eine feste Verklemmung nicht ein. Ein ausschliesslich s-förmig gebogenes Gebilde ist zur Erzielung eines Klemmsitzes nicht sehr geeignet.

Der Erfindung liegt die Aufgabe zugrunde, eine Hüftgelenkprothese der eingangs genannten Art derart auszubilden, dass einerseits die Krafteinleitung über einen ausgedehnten Bereich ohne punktuelle Belastung erfolgen kann – andererseits aber zu grosse radiale Spannungen im Femurbereich vermieden sind.

Diese Aufgabe ist durch eine Hüftgelenkprothese mit den im kennzeichnenden Teil des Anspruchs 1 angegebenen Merkmalen gelöst.

Die Erfindung beruht auf der Erkenntnis, dass der Markkanal im Bereich des Trochanter minor, in den die zusätzliche einseitig vorgesehene Erhebung des Schafts der erfindungsgemässen Hüftgelenkprothese eingreift, eine Krafteinleitung ermöglicht, die einen festen Sitz der Prothese bei begrenzten durch Keilwirkung verursachten Kräften sicherstellt. Gleichzeitig ist eine Drehsicherung gewährleistet und damit eine Auslockerung infolge von quer zur Mittelachse gerichteten Kräften verhindert. Die Einleitung von Belastungskräften erfolgt vorwiegend in einem Bereich unterhalb der zum Einlassen in den Trochanter minor bestimmten Erhebung, der eine vergrösserte Verjüngung aufweist, während die Aufgabe des weiteren distalen Schaftbereichs bevorzugt in der Überleitung von quer zur Prothesenlängsachse gerichteten Kräften besteht. Bezugspunkt für die sich hierbei ergebenden Momente ist ebenfalls derjenige Teil der Erhebung, welcher im kleinen Trochanter Platz findet.

Die Vergrösserung des Querschnitts im Bereich der maximalen Krümmung der Prothese dient bevorzugt zur Aufnahme einer Bohrung zur Fixierung, da hier ein vergrösserter Materialquerschnitt des Schaftes zur Verfügung steht. Zusätzlich befindet sich im Bereich des distalen Endes eine Gewindebohrung zur Fixation. Der Schaft der erfindungsgemässen Hüftgelenkprothese besteht vorzugsweise aus einer Titanlegierung oder aber aus mit Kohlenstoffasern verstärktem Kunststoff.

Die erfindungsgemässe Prothese unterscheidet sich damit grundsätzlich auch beispielsweise von einer aus der DE-C 2 059 381 bekannten Prothese, bei der eine Bohrung an einem Ansatz des Kragens vorgesehen ist. Hier befindet sich der Ansatz auf dem «Rücken» des Bereichs verhältnismässig starker Krümmung oder der Abbiegung. Eine Verankerung im Trochanter minor ist ausgeschlossen.

2

Das Einsetzen der Prothese ist dadurch erleichtert, dass der Querschnitt des Schaftes im Bereich der einseitigen Erhebung durch die physiologische Gestaltung dem Querschnitt kurz unterhalb des zur Aufnahme der Gelenkkugel dienenden Endes angepasst ist. Der abgerundete Querschnitt ist in diesem Bereich beidseitig abgeflacht, ovoid, ellipsenförmig oder oval ausgebildet.

Das Einsetzen lässt sich bei einer entsprechenden Gestaltung der Öffnung im proximalen Knochenende ohne weiteres dadurch erreichen, dass das Schaftende beim Einsetzen zunächst um einen Winkel von etwa 90° verdreht geführt wird, bis der Bereich der einseitigen Erhebung die äussere Knochenöffnung passiert hat. Mit dem Erreichen des Bereichs des Trochanter minor im Markkanal wird der Schaft entsprechend zurückgedreht, so dass der Schaftbereich mit grösstem Querschnitt an dem der Gelenkkugel nahen Ende sich in die Öffnung einfügt, wobei sich die Schulter im Bereich des Trochanter minor abstützt. Dadurch ist ein sicherer Sitz der Prothese gewährleistet. Da der Schaft bei anatomischer Gestaltung als Links- oder Rechtsprothese verhältnismässig kurz ausgebildet sein kann, treten beim Einbringen keine Probleme auf, und die Führung in die geeignete Winkelposition erfolgt gleichsam «von selbst», da nur in der jeweils korrekten Winkelposition ein weiteres Einführen ermöglicht ist.

Gemäss bevorzugten Weiterbildungen der Erfindung weist die Prothese zusätzlich eine geringe Krümmung in einer zu der Ebene grösserer Krümmung senkrechten Ebene auf – wie es aus der DE-A 2 851 598 bekannt ist, wobei der oder die Mittelpunkte der Krümmungsradien sich auf der der einseitigen Erhebung gegenüber angeordneten Seite des Schaftes befinden.

Andere vorteilhafte Weiterbildungen sind in den abhängigen Ansprüchen angegeben. Ein Ausführungsbeispiel der Erfindung wird nachfolgend näher beschrieben. Es zeigen:

Fig. 1 die erfindungsgemässe Prothese von der Körperachse her gesehen, in der AP-Ebene dargestellt.

Fig. 2 dieselbe Prothese von posterior her betrachtet (Zeichenebene ist die LM-Ebene) und

Fig. 3 und 4 Querschnitte durch die erfindungsgemässe Prothese in zwei verschiedenen Schnittebenen gemäss Figur 2, wobei Figur 3 in bezug auf Figur 4 um 90° versetzt dargestellt ist. Die Zeichnung ist etwa im Massstab 1:1 gehalten.

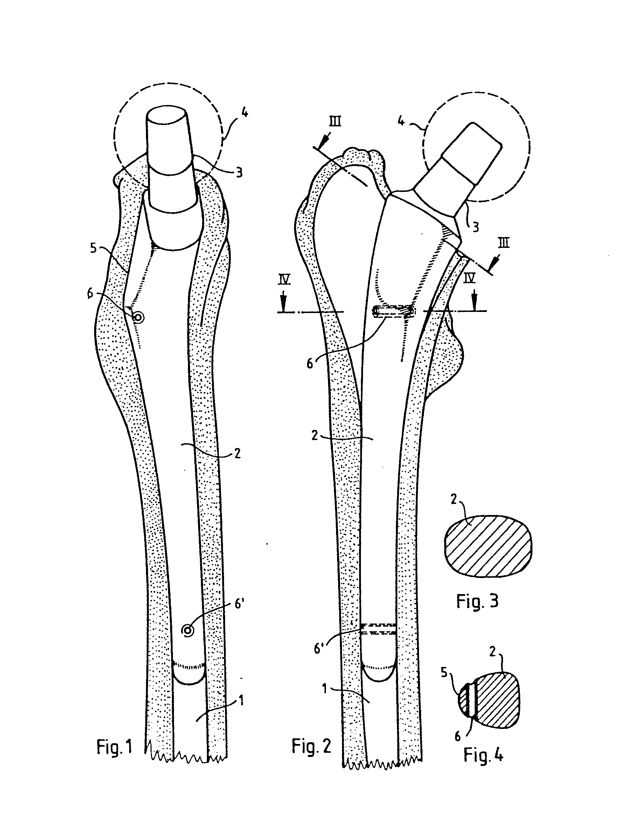

Der in den Markkanal des Femurs 1 eingesetzte Prothesenschaft 2 weist in seiner Ebene maximaler Krümmung (Darstellungsebene gemäss Figur 2 ist die LM-Ebene) einen Verlauf auf, bei dem die Krümmung vom proximalen zum distalen Ende hin abnimmt. Das proximale Ende trägt einen Ansatz 3, welcher zur Aufnahme einer (gestrichelt dargestellten) Gelenkkugel 4 dient.

Der Querschnitt des Prothesenschafts ist über seine gesamte Länge im wesentlichen gerundet, wobei das proximale Ende kurz unterhalb des Ansatzes 3 eine Kontur aufweist, wie sie in Figur 3 dargestellt ist.

Diese Kontur ist als oval oder ellipsenförmig anzusprechen. Zu seinem distalen Ende hin verringert sich dieser Querschnitt kontinuierlich, wobei – abgesehen von der noch zu beschreibenden einseitigen Erhebung – die abgeflachte Querschnittform im wesentlichen beibehalten wird. Die Querschnittsachse mit kleinerer Abmessung steht dabei senkrecht zur LM-Ebene, in der auch die Hauptkrümmung des Prothesenschaftes verläuft.

Wie aus Figur 1 ersichtlich ist, weist der Schaft noch eine weitere Krümmung mit grösserem Radius auf, deren Mittelpunkt sich im wesentlichen senkrecht über der Ebene befindet, in dem die grössere Krümmung verläuft – sie dient zur Anpassung an die besonderen Gegebenheiten des linken und rechten Femur.

Die Prothese kann sowohl aus körperverträglichen Metallegierungen als auch aus entsprechenden kohlenstoffaserverstärkten Kunststoffen bestehen, wobei die Werkstoffauswahl derart erfolgt, dass eine ausreichende Festigkeit gewährleistet ist.

Die erfindungsgemässe Prothese ist mit einer einseitigen Erhebung in Form einer Schulter 5 versehen, welche in ihrer Kontur dem Trochanter minor folgt. In Längsrichtung des Schaftes 2 auf der Oberfläche über den Bereich der Schulter verläuft die Kontur dabei derart, dass eine entsprechende Begrenzungslinie von einem Bereich, der mit der Mittelachse einen kleinen Winkel – entsprechend der normalen Verjüngung – bildet, zum Gipfel der Erhebung 5 hin in einen Bereich übergeht, der mit der Mittelachse einen vergrösserten Winkel bildet, wobei der Übergang kontinuierlich erfolgt. Diese Linie bildet die linke äussere Begrenzung in Figur 1 und verläuft zum Scheitelpunkt der Erhebung 5 im Bereich der Bohrung 6 im wesentlichen konkav. Die Schulter 5 weist im Bereich ihrer höchsten Erhebung einen kammartigen Gipfelbereich auf, der sich in Umfangsrichtung erstreckt. Vom Gelenkende des Schaftes her folgt die schulterförmige Erhebung eher dem Querschnitt mit schärfer verrundeten Eckbereichen mit eingeschlossenen, im wesentlichen ebenen Flächenteilen gemäss Figur 3, während der nach unten an den Kamm anschliessende Bereich sich – ausgehend von dem in Figur 4 im Bereich der Bohrung 6 dargestellten Profil – zum Ende hin einer gleichmässigen Verrundung nähert.

Der Querschnitt in diesem Bereich ist in Figur 4 dargestellt, wobei ersichtlich ist, dass eine Annäherung an die Kontur des Querschnitts im Bereich der Linie III–III in Figur 2 gegeben ist, so dass dieser Querschnittsbereich gemäss Figur 4 ohne Schwierigkeiten eine Öffnung passieren kann, welche den Querschnitt gemäss Figur 3 durchlässt. Es ist dabei lediglich eine Drehung um die Längsachse um einen vorgegebenen Winkel von im wesentlichen 90° notwendig. Da die Kontur des Schaftes gerundet ohne Stufen ausgebildet ist, ergibt sich eine nahezu selbsttätige Führung beim Einbringen in den Markraum.

Der Bereich des verstärkten Materialquerschnitts der Schulter 5 dient zur Aufnahme einer Gewindebohrung 6, mit der der Schaft durch eine Schraube in der gewählten Position fixierbar ist. Diese Fixierung bildet eine zusätzliche Sicherung, da durch die den Trochanter minor ausfüllende Formung der Prothese deren stabiler Sitz sichergestellt ist. Die Bohrung 6 verläuft dabei in der Ebene der Abbiegung oder Krümmung und ist im wesentlichen senkrecht zur Mittelachse des Schafts gerichtet.

Der Bereich, wie er dem in Figur 4 wiedergegebenen Querschnitt entspricht, schneidet die Schulter 5 im Bereich ihrer höchsten Erhebung. Ohne diese Schulter würde der im gelenknahen Bereich gekrümmte (Curvation) Schaft sich zu seinem anderen Ende hin stetig verjüngen. Er würde in der Ebene dieser Krümmung stets eine grössere Erstreckung haben als senkrecht dazu. Die schulterförmige Erhebung ist dieser Grundstruktur hinzugefügt und ragt aus der Ebene der Krümmung heraus, wobei in Längsrichtung des Schaftes die (zusätzliche) Steigung geringer ist als in Umfangsrichtung. Die Schulter bildet also eine zusätzliche Ausbauchung des sonst im wesentlichen eine ebenmässige Kontur aufweisenden Prothesenschaftes. Die Schulter ragt in einem Bereich des Schaftes seitlich hervor, der im wesentlichen in der Mitte der Innenkurve des stärker gekrümmten Bereichs angeordnet ist.

Der Schnitt gemäss Figur 4 geht durch den Gipfelbereich (Kammlinie) der als Schulter 5 ausgebildeten Erhebung. Durch die (zusätzliche) Schulter wird der Querschnitt des verjüngten Schafts einseitig in der Weise vergrössert, dass die Richtung der grössten Querschnittserstreckung hier nicht mehr in der Krümmungsebene verläuft, sondern in einer 90°-Richtung dazu (in Figur 4 also waagerecht statt senkrecht – die Darstellung von Figur 4 ist in bezug auf Figur 3 bereits um 90° gedreht). Wenn also der Querschnitt des Schafts gemäss Figur 4 die obere für den Prothesenquerschnitt gemäss Figur 3 vorbereitete Öffnung passieren soll, muss der Schaft beim Einführen des Bereichs gemäss Figur 3 um 90° gedreht sein, um dann in der (fast) endgültig eingeführten Position seine Normallänge einnehmen zu können. Bei diesem Drehen in die Normalposition gelangt die Schulter in eine Erweiterung der aus Corticalis bestehenden äusseren Knochenstruktur hinein, die als «Trochanter minor» bezeichnet wird. Damit wirkt die Schulter buchstäblich wie ein Zapfen entsprechend einem einseitigen Bajonettverschluss. Die Masse des Schaftes werden dabei den Dimensionen des menschlichen Femurs angepasst.

Eine entsprechende Gewindebohrung ist auch im Bereich des distalen Schaftendes vorgesehen, was durch die gestrichelte Bohrung 6' angedeutet ist. Diese Bohrung verläuft in der Richtung lateral-medial, da diese Richtung durch das bei der Implantation benutzte Zielgerät leicht zu erreichen ist. Die entsprechenden Fixationsschrauben durchqueren den Femur vollkommen und dienen der zusätzlichen Fixierung des Schaftes.

Die in den Figuren 1 bis 4 wiedergegebene Prothese ist für die Anwendung links gedacht. Sie kann wegen der im Bereich des Trochanter minor ohnehin unsymmetrischen und damit unterschiedlichen Gestaltung für links und rechts durch die in Figur 1 wiedergegebene, in der AP-Ebene verlaufende Krümmung mit grossem Radius den anatomischen Voraussetzungen im linken oder rechten Femur zusätzlich angepasst werden, so dass ein optimaler Sitz gewährleistet ist.

Der Bereich maximaler Krafteinleitung bei der erfindungsgemässen Prothese liegt im sich vom distalen Ende zur einseitigen Schulter 5 hin erweiternden Oberflächenbereich. Der sich ergebende Keilwinkel ist dabei so bemessen, dass die radial gerichteten Kräfte begrenzt sind. Der Aufsitz der Prothese und damit die Einleitung von vertikalen Belastungskräften ist über einen so grossen Bereich verteilt, dass Knochenresorption verursachende Mikrobewegungen ausgeschlossen sind. Die Markraumöffnung braucht nicht in aufwendiger Weise einer komplizierten Formgebung unterworfen zu werden, da die erfindungsgemässe Prothese von den vorgefundenen physiologischen Voraussetzungen Gebrauch macht.

Die Länge des Prothesenhalses kann – entsprechend den individuellen Voraussetzungen – bei der Herstellung variiert werden.

**Patentansprüche**

1. Kragenlose Hüftgelenkprothese zur zementfreien Verankerung mit einem Schaft (2), der in der Nähe seines zur Aufnahme der Gelenkkugel (4) bestimmten Endes eine Richtungsänderung durch Abbiegung oder Krümmung aufweist, wobei der abgerundete Schaftquerschnitt senkrecht zu der Lateral-Medial-Ebene, in der die Abbiegung oder Krümmung verläuft, eine geringere Querabmessung aufweist als in der Ebene, dadurch gekennzeichnet, dass am Schaft (2) im Bereich der Krümmung oder Abbiegung eine aus der Lateral-Medial-Ebene einseitig herausragende, zur Abstützung im Bereich des Trochanter minor bestimmte schulterförmige Erhebung (5) vorgesehen ist, wobei die schulterförmige Erhebung (5) Steigungen aufweist, die in Längsrichtung des Schaftes geringer sind als in Querrichtung, wobei die Steigungen bezogen sind auf die Form des im wesentlichen ovalen, sich gleichmässig verjüngenden Schafts mit der Abbiegung oder Krümmung im Halsbereich.

2. Hüftgelenkprothese nach Anspruch 1, dadurch gekennzeichnet, dass die schulterförmige Erhebung (5) eine in Querrichtung verlaufende konvexe Kammlinie aufweist, während die sich in Längsrichtung des Schaftes zur Kammlinie hin erstreckende Aussenkontur beidseitig der Erhebung konkav ausgebildet ist.

3. Hüftgelenkprothese nach einem der vorangehenden Ansprüche, dadurch gekennzeichnet, dass der Schaft (2), ausgehend von seinem zur Aufnahme der Gelenkkugel (4) dienenden Ende, eine Verjüngung aufweist, wobei die Querschnittskontur im Bereich der schulterförmigen Erhebung (5) demjenigen Bereich maximalen Querschnitts angepasst ist, der sich unmittelbar bei dem die Gelenkkugel aufnehmenden oder die Gelenkkugel bildenden Ende befindet.

4. Hüftgelenkprothese nach Anspruch 3, dadurch gekennzeichnet, dass die Richtung maximaler Erstreckung der Querschnittskontur (Figur 4) im Bereich der schulterförmigen Erhebung (5) und die Richtung maximaler Erstreckung in demjenigen Bereich maximalen Querschnitts (Figur 3), der sich unmittelbar bei dem die Gelenkkugel aufnehmenden oder die Gelenkkugel bildenden Ende befindet, in axialer Richtung der Prothese gesehen, einen Winkel miteinander bilden.

5. Hüftgelenkprothese nach Anspruch 4, dadurch gekennzeichnet, dass die Richtungen maximaler Erstreckung als sich räumlich nicht berührende Geraden im wesentlichen senkrecht zueinander gerichtet sind.

6. Hüftgelenkprothese nach einem der Ansprüche 4 und 5, dadurch gekennzeichnet, dass die Querschnittskontur (Figur 4) im Bereich der schulterförmigen Erhebung (5) die Fläche des Bereichs maximalen Querschnitts (Figur 3), der sich unmittelbar bei dem die Gelenkkugel aufnehmenden oder die Gelenkkugel bildenden Ende befindet, bei deckungsgleicher Ausrichtung nicht überragt.

7. Hüftgelenkprothese nach einem der vorangehenden Ansprüche, dadurch gekennzeichnet, dass die schulterförmige Erhebung (5) eine Durchbohrung (6) aufweist, welche im wesentlichen senkrecht zur Mittelachse gerichtet ist und parallel zur Ebene, in der die Abbiegung oder Krümmung verläuft, den Bereich der Erhebung schneidet.

8. Hüftgelenkprothese nach einem der vorangehenden Ansprüche, dadurch gekennzeichnet, dass in der Nähe des vom Anschluss für die Gelenkkugel entfernten Endes eine quer zur Längsachse der Prothese verlaufende Bohrung (6') vorgesehen ist.

9. Hüftgelenkprothese nach Anspruch 8, dadurch gekennzeichnet, dass die Bohrung (6) ein Maschinengewinde aufweist.

10. Hüftgelenkprothese nach einem der vorangehenden Ansprüche, dadurch gekennzeichnet, dass der Schaft (2) eine weitere, aber geringer ausgebildete Krümmung um einen Punkt als Mittelpunkt aufweist, welcher sich an der der einseitigen schulterförmigen Erhebung (5) gegenüberliegenden Seite befindet.

## Claims

1. Collarless artificial hip-joint intended for fixing without cement, having a shaft (2) which close to the end will receive the ball (4) of the joint has a change of direction formed by a bend or curve, and the rounded shaft cross-section in which the bend or curve occurs, has a smaller transverse dimension perpendicular to the lateral-medial plane than in that plane, characterised in that on the shaft (2) in the region of the curve or bend is a shoulder-like lump projecting on one side of the lateral-medial plane to provide support in the region of the lesser trochanter, the shoulder-like lump (5) having slopes which are smaller in the longitudinal direction of the shaft than in the transverse direction, the slopes being relative to the shape of the generally oval shaft which tapers uniformly with the bend or curve in the throat region.

2. Artificial hip-joint according to claim 1, characterised in that the shoulder-like lump (5) has a convex ridge line extending in the transverse direction, while the external contour extending in the longitudinal direction of the shaft up to the ridge line on both sides of the lump is of concave shape.

3. Artificial hip-joint according to one of the preceding claims, characterised in that the shaft (2), starting from its end for receiving the ball (4) of the joint, has a taper, so that the contour of the cross-section in the region of the shoulder-like lump (5) is matched to that region of maximum cross-section which is to be formed immediately next to the end which receives or forms the ball of the joint.

4. Artificial hip-joint according to claim 3, characterised in that the direction of the maximum dimension of the cross-sectional contour (Fig. 4) in the region of the shoulder-like lump (5), and the direction of the maximum dimension in the region of the maximum cross-section (Fig. 3), occurring directly next to the end which receives or forms the ball of the joint, seen in the axial direction of the prothesis, form an angle to each other.

5. Artificial hip-joint according to claim 4, characterised in that the directions of maximum dimension form straight lines which do not meet in space and lie substantially perpendicular to each other.

6. Artificial hip-joint according to claim 4 or 5, characterised in that the cross-sectional contour (Fig. 4) in the region of the shoulder-like lump (5) does not extend beyond the surface of the region of maximum cross-section (Fig. 3) which occurs immediately next to the end which receives or forms the ball of the joint, when placed congruently.

7. Artificial hip-joint according to one of the preceding claims, characterised in that the shoulder-like lump (5) has a bore (6) through it which is substantially perpendicular to the main axis and cuts through in the region of the lump parallel to the plane in which the bend or curve runs.

8. Artificial hip-joint according to one of the preceding claims, characterised in that a bore (6') is provided extending transverse to the longitudinal axis of the prothesis and close to the end remote from the connection for the ball of the joint.

9. Artificial hip-joint according to claim 8 characterised in that the bore (6) is threaded.

10. Artificial hip-joint according to one of the preceding claims, characterised in that the shaft (2) has a further, but less pronounced curve about a point forming the centre on the side opposite to the one-sided shoulder-like lump (5).

**Revendications**

1. Prothèse d'articulation de la hanche sans collerette, pour ancrage sans ciment, comprenant une tige (2) qui présente près de son extrémité destinée à recevoir la rotule d'articulation formant tête fémorale, un changement de direction par devers ou courbure, la section droite arrondie de la tige présentant une plus petite dimension transversale perpendiculairement au plan latéro-médial, dans lequel s'étend le devers ou la courbure, que dans ce plan, caractérisée en ce que la région de la tige (2) où se trouve la courbure ou le devers présente une élévation ou bosse (5) en forme d'épaulement, qui fait saillie unilatéralement et est destinée à servir d'appui dans la région du petit trochanter, cette bosse (5) en forme d'épaulement possédant des pentes plus faibles dans le sens longitudinal de la tige que dans le sens tranversal, les pentes étant considérées par rapport à la forme essentiellement ovale en section et s'amincissant régulièrement de la tige, avec le devers ou la courbure dans la région du col.

2. Prothèse d'articulation de la hanche selon la revendication 1, caractérisée en ce que la bosse (5) en forme d'épaulement présente une ligne de crête convexe s'étendant en direction transversale, tandis que le contour extérieur s'étendant dans le sens longitudinal de la tige vers la ligne de crête est concave des deux côtés de la bosse.

3. Prothèse d'articulation de la hanche selon une des revendications précédentes, caractérisée en ce que la tige (2) présente un amincissement à partir de son extrémité destinée à recevoir la rotule d'articulation (4), avec adaptation du contour de section droite, dans la région de la bosse (5) en forme d'épaulement, à la région de section droite maximale située à proximité immédiate de l'extrémité recevant la rotule d'articulation ou constituant la rotule d'articulation.

4. Prothèse d'articulation de la hanche selon la revendication 3, caractérisée en ce que la direction d'étendue maximale du contour de section droite (figure 4) dans la région de la bosse (5) en forme d'épaulement et la direction d'étendue maximale dans la région de section droite maximale (figure 3) située à proximité immédiate de l'extrémité recevant la rotule d'articulation ou constituant la rotule d'articulation, forment un angle entre elles dans le sens de l'axe de la prothèse.

5. Prothèse d'articulation de la hanche selon la revendication 4, caractérisée en ce que les directions d'étendue maximale sont orientées suivant des droites essentiellement perpendiculaires, mais qui ne se touchent pas.

6. Prothèse d'articulation de la hanche selon la revendication 4 ou 5, caractérisée en ce que le contour de section droite (figure 4) dans la région de la bosse (5) en forme d'épaulement ne dépasse pas l'aire de la région de section droite maximale (figure 3) située à proximité immédiate de l'extrémité recevant la rotule d'articulation ou constituant la rotule d'articulation, lorsqu'ils sont mutuellement orientés de manière à se recouvrir.

7. Prothèse d'articulation de la hanche selon une des revendications précédentes, caractérisée en ce que la bosse (5) en forme d'épaulement présente un perçage (6) qui est essentiellement orienté perpendiculairement à l'axe médian et qui coupe la région de la bosse parallèlement au plan dans lequel s'étend le devers ou la courbure.

8. Prothèse d'articulation de la hanche selon une des revendications précédentes, caractérisée en ce qu'elle possède un perçage (6') près de l'extrémité opposée à celle prévue pour le raccordement de la rotule d'articulation, perçage qui est orienté transversalement à l'axe longitudinal de la prothèse.

9. Prothèse d'articulation de la hanche selon la revendication 8, caractérisée en ce que le perçage (8) présente un taraudage machine.

10. Prothèse d'articulation de la hanche selon l'une des revendications précédentes, caractérisée en ce que la tige (2) présente une courbure supplémentaire, mais plus faible, autour d'un point, formant centre de courbure, qui est situé du côté opposé à celui où se trouve la bosse unilatérale (5) en forme d'épaulement.

Fig. 1

Fig. 2

Fig. 3

Fig. 4